# FASCICULE DE BREVET EUROPEEN

(11) **EP 0 842 652 B1**
(45) Date de publication et mention de la délivrance du brevet: **03.07.2002**
(21) Numéro de dépôt: 97402295.6
(22) Date de dépôt: 01.10.1997
(51) Int. Cl.: A61K 7/00

(54) **Nanoémulsion à base de lipides amphiphiles non-ioniques et cationiques et utilisation**
Nanoemulsion auf Basis von nichtionischen und kationischen, amphiphilen Lipiden und ihre Verwendung
Nanoemulsion based on non-ionic and cationic amphiphilic lipids and their use

(30) Priorité: 15.11.1996 FR 9613978; 18.03.1997 FR 9703281
(43) Date de publication de la demande: 20.05.1998
(73) Titulaire: L'OREAL, 75008 Paris (FR)
(72) Inventeur: Restle, Serge, 95390 Saint Prix (FR); Cauwet-Martin, Danièle, 75011 Paris (FR)
(74) Mandataire: Andral, Christophe André Louis

(56) Documents cités:
- EP-A- 0 334 777
- EP-A- 0 433 131
- EP-A- 0 433 132
- EP-A- 0 490 053
- FR-A- 2 730 932

## Description

La présente invention a trait à une nanoémulsion huile-dans-eau dont les globules d'huile ont une taille moyenne inférieure à 150 nm et comprenant une phase lipidique amphiphile à base de lipides amphiphiles non-ioniques liquides à une température ambiante inférieure à 45°C et de lipides amphiphiles cationiques ainsi qu'à leur utilisation en application topique notamment en cosmétique et en dermopharmacie.

Les émulsions huile-dans-eau sont bien connues dans le domaine de la cosmétique et de la dermopharmacie notamment pour la préparation de produits cosmétiques tels que des lotions, des toniques, des sérums, des eaux de toilette.

Cependant, la présence de concentrations importantes d'huiles végétales, animales ou minérales dans des compositions rend leur formulation difficiles. En effet, les compositions sont généralement instables au stockage et les propriétés cosmétiques sont insuffisantes. En particulier, l'application de telles compositions sur les cheveux entraîne un toucher gras, une difficulté de rinçage. De plus, les cheveux séchés sont sans volume et ont un toucher chargé.

On connaît dans FR2730932 des émulsions huile-dans-eau ayant des globules d'huile dont la taille moyenne est inférieure à 100 nm comprenant une phase lipidique amphiphile comprenant au moins un lipide amphiphile non-ionique liquide à une température ambiante inférieure à 45°C et dont le rapport pondéral de la quantité d'huile sur la quantité de phase lipidique amphiphile est compris entre 2 et 10.
Par ailleurs, EP490053 décrit des microémulsions contenant un tensioactif cationique, une huile et un tensioactif non ionique de HLB compris entre 5 et 12.

On connaît dans l'état de la technique des nanoémulsions comprenant une phase lipidique amphiphile constituée de phospholipides, d'un lipide cationique, d'eau et d'un filtre solaire hydrophobe.
Elles sont obtenues par un procédé d'homogénéisation à haute pression. Ces émulsions présentent l'inconvénient d'être instables au stockage aux températures traditionnelles de conservation à savoir entre 0 et 45°C. Elles conduisent à des compositions jaunes et produisent des odeurs de rance qui se développent après quelques jours de conservation. De plus, ces émulsions n'apportent pas de bonnes propriétés cosmétiques. Elles sont décrites dans la revue « DCI » d'avril 1996, pages 46-48.

La demanderesse a découvert, de façon inattendue, de nouvelles nanoémulsions dont les globules d'huile ont une taille moyenne inférieure à 150 nm, stables au stockage entre 0 et 45°C après au moins un mois. Les nanoémulsions conformes à l'invention sont préparées à des températures entre 20 et 45°C et sont compatibles avec des actifs thermosensibles. Elles peuvent contenir des quantités importantes d'huile. Elles peuvent notamment contenir des quantités importantes de parfum et améliorer leur rémanence. Elles favorisent également la pénétration des actifs dans les couches superficielles de la peau et le dépôt d'actif sur les fibres kératiniques telles que les cheveux. Les cheveux traités avec ces nanoémulsions sont brillants sans avoir un toucher ou un aspect gras, ils se démêlent facilement, sont plus doux et plus nerveux.

La présente invention a pour objet des nanoémulsions huile-dans-eau ayant des globules d'huile dont la taille moyenne est inférieure à 150 nm, caractérisée par le fait qu'elles comprennent une phase lipidique amphiphile comprenant au moins un lipide amphiphile non-ionique liquide à une température ambiante inférieure à 45°C et au moins un lipide amphiphile cationique et que le rapport pondéral de la quantité d'huile sur la quantité de phase lipidique amphiphile est compris entre 2 et 10 et de préférence entre 3 et 6.

Les lipides amphiphiles non-ioniques de l'invention sont préférentiellement choisis parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée. On peut également utiliser les mélanges des composés ci-dessus.

Les tensioactifs siliconés utilisables selon l'invention sont des composés siliconés comportant au moins une chaîne oxyéthylénée -OCH₂CH₂- et/ou oxypropylénée - OCH₂CH₂CH₂-. Comme tensioactifs siliconés pouvant être utilisés selon la présente invention, on peut citer ceux décrits dans les documents US-A-5364633 et US-A-5411744.

De préférence, le tensioactif siliconé utilisé selon la présente invention est un composé de formule (I) 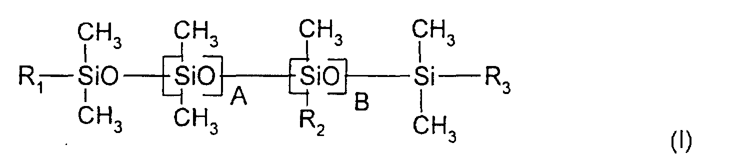 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 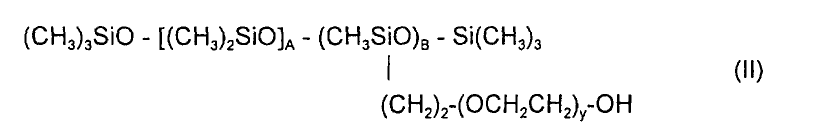 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III):

HO - (OCH₂CH₂)_{y}-(CH₂)₃ - [(CH₃)₂SiO]_{A'} - (CH₂)₃ - (OCH₂CH₂)_{y} - OH (III)

dans laquelle A' et y sont des nombres entiers allant de 10 à 20.

On peut utiliser comme composés de l'invention ceux commercialisés par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le composé Q4-3667 est un composé de formule (III) où A est 15 et y est 13.

Parmi les lipides amphiphiles non ioniques, on peut plus particulièrement citer, à titre d'exemple :
- l'isostéarate de polyéthylèneglycol de poids moléculaire 400,
- l'isostéarate de diglycéryle,
- le laurate de polyglycérol comportant 10 unités de glycérol,
- l'oléate de sorbitane,
- l'isostéarate de sorbitane,
- le cocoate d'α-butylglucoside ou le caprate d'α-butylglucoside.

Les lipides amphiphiles cationiques, utilisés dans les nanoémulsions de l'invention, sont choisis, de préférence, dans le groupe formé par les sels d'ammonium quaternaire, les amines grasses et leurs sels.

Les sels d'ammonium quaternaires sont par exemple :
- ceux qui présentent la formule générale (IV) suivante : 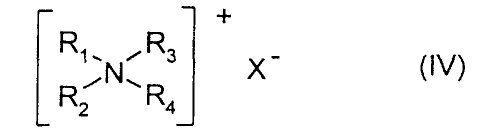 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (V) suivante : 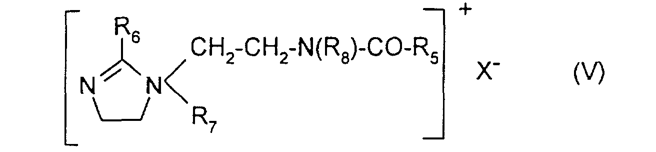 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (VI) : 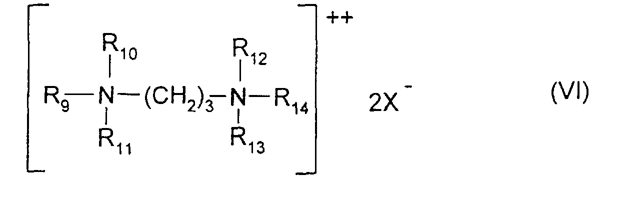 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (VII) suivante : 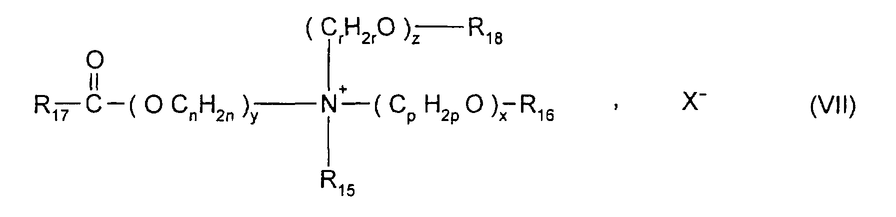 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
   - le radical 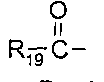
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 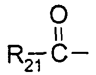
   - les radicaux R₂₂ hydrocarbonés en C₁-C₆ linéaires ou ramifiés, saturés ou insaturés,
   - l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 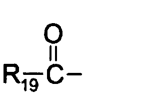
   - les radicaux méthyle, éthyle ou hydrocarbonés en C₁₄-C₂₂
   - l'atome d'hydrogène ;
- R₁₈ est choisi parmi :
   - le radical 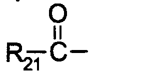
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés. Avantageusement, les radicaux hydrocarbonés sont linéaires.

On peut citer par exemple les composés de formule (VII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Selon l'invention, le chlorure de béhényltriméthylammonium est le sel d'ammonium quaternaire le plus particulièrement préféré.

Les lipides cationiques amphiphiles sont présents dans les nanoémulsions de l'invention, de préférence, dans des concentrations allant de 1 à 60% en poids et plus particulièrement de 10 à 50 % en poids par rapport au poids total de la phase lipidique amphiphile.

Les lipides cationiques amphiphiles sont présents dans les nanoémulsions de l'invention, de préférence, dans des concentrations allant de 0,1 à 10% en poids par rapport au poids total de la nanoémulsion.

Les nanoémulsions conformes à l'invention comportent une quantité d'huile allant de préférence, de 5 à 40% en poids par rapport au poids total de l'émulsion et plus particulièrement de 8 à 30% en poids.

Les huiles pouvant être utilisées dans les nanoémulsions de l'invention sont choisies préférentiellement dans le groupe formé par :
- les huiles animales ou végétales formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane et l'huile de paraffine ;
- des carbures halogénés, notamment des fluorocarbures tels que des fluoramines par exemple la perfluorotributylamine, des hydrocarbures fluorés, par exemple le perfluorodécahydronaphtalène, des fluoroesters et des fluoroethers ;
- des esters d'acide minéral et d'un alcool ;
- des éthers et des polyéthers ;
- des silicones en mélange avec au moins l'une des huiles définies ci-dessus, par exemple le décamethylcyclopentasiloxane ou le dodécaméthylcyclohexasiloxane.

Les nanoémulsions conformes à la présente invention peuvent contenir des additifs pour améliorer, si nécessaire, la transparence de la formulation.

Ces additifs sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

Les additifs tels que ceux cités ci-dessus sont présents dans les nanoémulsions de l'invention dans des concentrations allant , de préférence, de 1 à 30% en poids par rapport au poids total de l'émulsion.

En outre, l'utilisation des alcools tels que définis ci-dessus, à des concentrations supérieures ou égales à 5% en poids et de préférence supérieure à 15%, permet d'obtenir des émulsions sans conservateur.

Les nanoémulsions de l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines telles que la vitamine E et ses dérivés, les provitamines telles que le panthénol, les humectants, les filtres solaires siliconés ou non, des agents tensioactifs, des agents conservateurs, des séquestrants, des adoucissants, des colorants, des agents modificateurs de viscosité, des agents modificateurs de mousse, des stabilisateurs de mousse, des agents nacrants, des pigments, des agents hydratants, des agents antipelliculaires, des agents antiséborrhéiques, des protéines, des silicones, des céramides, des pseudocéramides, des acides gras à chaînes linéaires ou ramifiées en C₁₆-C₄₀ tels que l'acide méthyl-18 eicosanoique, des épaississants, des plastifiants, des hydroxyacides, des électrolytes, des polymères en particulier cationiques et des parfums.

Parmi les épaississants utilisables, on peut citer les dérivés de cellulose tels que l'hydroxyméthylpropylcellulose, les alcools gras tels que les alcools stéarylique, cétylique, béhénique, les dérivés d'algues tels que le satiagum, des gommes naturelles telles que l'adragante et des polymères synthétiques tels que les mélanges d'acides polycarboxyvinyliques commercialisés sous la dénomination CARBOPOL® par la société GOODRICH et le mélange de copolymères acrylate de Na/acrylamide commercialisé sous la dénomination HOSTACERIN® PN 73 par la société HOECHST.

Les globules d'huile des nanoémulsions de l'invention, ont de préférence une taille moyenne allant de 30 à 150 nm, plus préférentiellement de 40 à 100 nm et encore plus particulièrement de 50 à 80 nm.

Les nanoémulsions de l'invention peuvent être obtenues par un procédé, caractérisé par le fait qu'on mélange la phase aqueuse et la phase huileuse , sous agitation vive, à une température ambiante inférieure à 45°C puis qu'on effectue une homogénéisation haute pression à une pression supérieure à 10⁸ Pa et de préférence allant de 12.10⁷ à 18.10⁷ Pa. Un tel procédé permet de réaliser, à température ambiante, des nanoémulsions compatibles avec des composés actifs thermosensibles, et pouvant contenir des quantités importantes d'huiles et notamment des parfums qui renferment des corps gras, sans les dénaturer.

Un autre objet de l'invention consiste en une composition à usage topique telle qu'une composition cosmétique ou dermopharmaceutique, caractérisée par le fait qu'elle est constituée par une nanoémulsion telle que définie précédemment ou qu'elle comprend une telle nanoémulsion. L'invention concerne plus particulièrement les compositions capillaires.

Les compositions conformes à l'invention peuvent être utilisées pour le lavage, le nettoyage et le démaquillage des matières kératiniques telles que les cheveux, la peau, les cils, les sourcils, les ongles, les muqueuses.

Les compositions de l'invention peuvent plus particulièrement se présenter sous forme de shampooings, d'après-shampooings à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage.

Les compositions peuvent être également des lotions de mise en plis, des lotions pour le brushing, des compositions de fixation (laques) et de coiffage telles que par exemple des gels ou des mousses. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Un autre objet de l'invention est l'utilisation des nanoémulsions telles que définies ci-dessus comme base de produits de soin et/ou de maquillage et/ou démaquillage pour la peau et/ou le visage et/ou le cuir chevelu et/ou les cheveux et/ou les ongles et/ou les cils et/ou les sourcils et/ou les muqueuses (par exemple les lèvres), tels que des lotions, des sérums, des laits, des crèmes, des eaux de toilette.

Enfin, l'invention porte également sur un procédé non-thérapeutique de soin de la peau, des cheveux, des cils, des sourcils, des ongles, des muqueuses ou du cuir chevelu, caractérisé par le fait qu'on applique sur la peau, les cheveux, les cils, les sourcils, les ongles, les muqueuses ou le cuir chevelu une nanoémulsion telle que définie ci-dessus.

Les exemples qui suivent, permettront de mieux comprendre l'invention, sans toutefois présenter un caractère limitatif.

### EXEMPLES

Pour les exemples 1 et 4, le mode opératoire suivant est mis en oeuvre :
- dans une première phase A, on homogénéise les lipides amphiphiles non ioniques et cationiques avec l'huile et les actifs et adjuvants lipophiles à une température d'environ 45°C ;
- dans une seconde phase B, on dissout les actifs et adjuvants hydrophiles à une température de 20 à 30°C ;
- puis, on mélange les phases A et B à l'aide d'un homogénéisateur à turbine puis on homogénéise à l'aide d'un homogénéisateur à haute pression du type Soavi-Niro à une pression de 1500 bars, en 7 passages en maintenant la température du produit en dessous d'environ 35°C.

### EXEMPLE 1 : Nanoémulsion d'huile d'avocat

### Première phase :

- Isostéarate de PEG-400, vendu par la société UNICHEMA 4,5 %
- Chlorure de béhényltriméthylammonium (lipide amphiphile cationique) 0,5 %
- Huile d'avocat 20 %
- Ethanol absolu non dénaturé 15 %

### Deuxième phase :

- Eau déminéralisée 54,7 %
- Glycérine 5 %

On obtient une émulsion dont la taille des globules d'huile est d'environ 63 nm.

### EXEMPLE 2 : Soin capillaire non rincé

- Mélange de polyacrylamide, d'isoparaffine en C₁₃-C₁₄ et de laureth-7 vendu sous la dénomination SEPIGEL 305 par la société SEPPIC 0,9 g
- Nanoémulsion de l'exemple 1 15 g
- Conservateur, parfum qs
- HCl qs pH 6
- Eau qsp 100 g

Les cheveux traités avec cette composition sont faciles à démêler et ont un toucher naturel et non gras.

### EXEMPLE 3 : Après-shampooing rincé

- Alcool cétylstéarylique 4 g
- Mélange de myristate/palmitate/stéarate de myristyle cétyle et stéaryle vendu sous la dénomination Blanc de baleine synthétique par la société LASERSON 1 g
- Nanoémulsion de l'exemple 1 10 g
- Chlorure de béhényltriméthylammonium 3 g
- Conservateur, parfum qs
- Eau qsp 100 g

Les cheveux traités avec cette composition sont faciles à démêler, doux et brillants.

### EXEMPLE 4 : Nanoémulsion d'huile d'avocat

### Première phase :

- Isostéarate de PEG-400, vendu par la société UNICHEMA 4,5 %
- Méthosulfate de distéaryl éthyl hydroxyéthyl ammonium (lipide amphiphile cationique) 0,5 %
- Huile d'avocat 20 %
- Ethanol absolu non dénaturé 15 %

### Deuxième phase :

- Eau déminéralisée 54,7 %
- Glycérine 5 %

On obtient une émulsion particulièrement fluide dont la taille des globules est de l'ordre de 50nm.

### EXEMPLE 5 : Shampooing

- Lauryléthersulfate de sodium (C₁₂/C₁₄ à 70/30) à 2,2 moles d'oxyde d'éthylène en solution aqueuse à 28% de MA 17 gMA
- Cocoylbétaïne (DEHYTON AB 30) 2,5 gMA
- Nanoémulsion de l'exemple 4 7,5 g
- Monoisopropanolamide de coprah 3 g
- Distéarate d'éthylèneglycol 3 g
- Parfum, conservateur qs
- NaOH qs pH 7,1
- Eau déminéralisée qsp 100 g

Cette composition selon l'invention présente un excellent pouvoir moussant. Les cheveux traités avec cette composition sont faciles à démêler, doux et brillants.

## Revendications

1. Nanoémulsion huile-dans-eau dont les globules d'huile ont une taille moyenne inférieure à 150 nm comprenant une phase lipidique amphiphile, **caractérisée par le fait que** ladite phase lipidique amphiphile comprend au moins un lipide amphiphile non-ionique liquide à une température ambiante inférieure à 45°C et au moins un lipide amphiphile cationique et que le rapport pondéral de la quantité d'huile sur la quantité de phase lipidique amphiphile est compris entre 2 et 10 et de préférence entre 3 et 6.

2. Nanoémulsion selon la revendication 1, **caractérisée par le fait que** le lipide amphiphile non-ionique est choisi parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée, et leurs mélanges.

3. Nanoémulsion selon l'une quelconque des revendications 1 ou 2, **caractérisée par le fait que** le lipide amphiphile cationique est choisi dans le groupe formé par les sels d'ammonium quaternaire et les amines grasses.

4. Nanoémulsion selon la revendication 3, **caractérisée par le fait que** les sels d'ammonium quaternaire sont choisis dans le groupe formé par :
- les sels d'ammonium quaternaire de formule générale (IV) suivante : 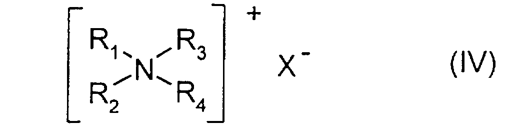 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium,
- les sels de diammonium quaternaire de formule (VI) : 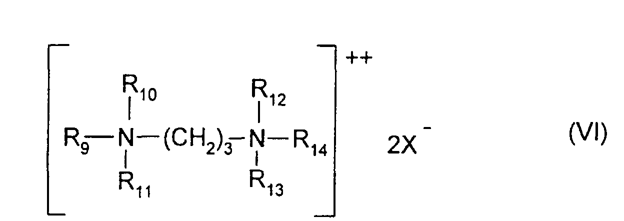 dans laquelle R₉ désigne un radical aliphatique comportant environ de 16 à 30 atomes de carbone, R₁₀, R₁₁, R₁₂, R₁₃ et R₁₄ sont choisis parmi l'hydrogène ou un radical alkyle comportant de 1 à 4 atomes de carbone, et X est un anion choisi dans le groupe des halogénures, acétates, phosphates, nitrates et méthylsulfates,
- les sels d'ammonium quaternaire contenant au moins une fonction ester.

5. Nanoémulsion selon l'une quelconque des revendications 1 à 4, **caractérisée par le fait que** le lipide amphiphile cationique est présent dans des concentrations allant de 1 à 60 % en poids par rapport au poids total de la phase lipidique amphiphile et de préférence de 10 à 50% en poids.

6. Nanoémulsion selon l'une quelconque des revendications 1 à 5, **caractérisée par le fait qu'**elle comprend une proportion d'huile allant de 5 à 40% en poids par rapport au poids total de l'émulsion.

7. Nanoémulsion selon l'une quelconque des revendications 1 à 6, **caractérisée par le fait que** l'huile est choisie dans le groupe formé par :
- les huiles animales ou végétales formées par des esters d'acides gras et de polyols ou bien les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone ;
- les huiles essentielles naturelles ou synthétiques ;
- les hydrocarbures ;
- les carbures halogénés ;
- les esters d'acide minéral et d'alcool ;
- les éthers et polyéthers ;
- les silicones en mélange avec au moins l'une des huiles définies ci-dessus.

8. Nanoémulsion selon l'une quelconque des revendications 1 à 7, **caractérisée par le fait qu'**elle contient un actif cosmétique ou dermopharmaceutique, hydrosoluble ou liposoluble.

9. Composition à usage topique, **caractérisée par le fait qu'**elle est constituée d'une nanoémulsion ou comprend une nanoémulsion selon l'une quelconque des revendications 1 à 8.

10. Utilisation d'une nanoémulsion telle que définie selon l'une quelconque des revendications 1 à 9 comme ou dans des produits de soin et/ou de lavage et/ou de maquillage et/ou démaquillage du corps et/ou du visage et/ou des muqueuses et/ou du cuir chevelu et/ou des cheveux et/ou des ongles, et/ou des cils et/ou des sourcils.

11. Procédé de traitement non-thérapeutique de la peau, des cheveux, des muqueuses, des ongles, des cils, des sourcils et/ou du cuir chevelu, **caractérisé par le fait qu'**on applique sur la peau, les cheveux, les muqueuses, les ongles, les cils, les sourcils ou le cuir chevelu une nanoémulsion selon l'une quelconque des revendications 1 à 9.

12. Procédé de préparation d'une nanoémulsion telle que définie selon l'une quelconque des revendications 1 à 9, **caractérisé par le fait qu'**on mélange la phase aqueuse et la phase huileuse , sous agitation vive, à une température ambiante inférieure à 45°C puis qu'on effectue une homogénéisation haute pression à une pression supérieure à 10⁸ Pa.

13. Procédé selon la revendication 12, **caractérisé par le fait que** la pression varie de 12.10⁷ à 18.10⁷ Pa.

## Patentansprüche

1. Öl-in-Wasser-Nanoemulsion, deren Ölkügelchen eine mittlere Größe unter 150 nm aufweisen und die eine amphiphile Lipidphase enthält, **dadurch gekennzeichnet, daß** die amphiphile Lipidphase mindestens ein bei einer Umgebungstemperatur unter 45 °C flüssiges, nichtionisches, amphiphiles Lipid und mindestens ein kationisches amphiphiles Lipid enthält, und dadurch, daß das Gewichtsverhältnis der Menge des Öls und der Menge der amphiphilen Lipidphase im Bereich von 2 bis 10 und vorzugsweise 3 bis 6 liegt.

2. Nanoemulsion nach Anspruch 1, **dadurch gekennzeichnet, daß** das nichtionische amphiphile Lipid unter den siliconhaltigen grenzflächenaktiven Stoffen, den Estern mindestens eines Polyols, das unter Polyethylenglykol mit 1 bis 60 Ethylenoxideinheiten, Sorbitan, Glycerin mit 2 bis 30 Ethylenoxideinheiten und Polyglycerinen mit 2 bis 15 Glycerineinheiten ausgewählt ist, und mindestens einer Fettsäure, die mindestens eine gesättigte oder ungesättigte, geradkettige oder verzweigte C₈₋₂₂-Alkylkette enthält, und deren Gemischen ausgewählt ist.

3. Nanoemulsion nach einem der Ansprüche 1 oder 2, **dadurch gekennzeichnet, daß** das kationische amphiphile Lipid unter den quartären Ammoniumsalzen und den Fettaminen ausgewählt ist.

4. Nanoemulsion nach Anspruch 3, **dadurch gekennzeichnet, daß** die quartären Ammoniumsalze ausgewählt sind unter:
- den quartären Ammoniumsalzen der folgenden allgemeinen Formel (IV): 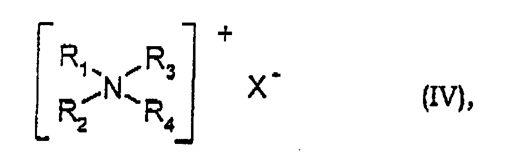
worin die Gruppen R₁ bis R₄, die identisch oder voneinander verschieden sein können, eine geradkettige oder verzweigte, aliphatische Gruppe mit 1 bis 30 Kohlenstoffatomen oder eine aromatische Gruppe, wie Aryl oder Alkylaryl, bedeuten und X ein Anion ist, das unter den Halogeniden, Phosphaten, Acetaten, Lactaten, C₂₋₆-Alkylsulfaten und Alkyl- oder Alkylarylsulfonaten ausgewählt ist;
- den quartären Ammoniumsalzen von Imidazolinium;
- den quartären Diammoniumsalzen der Formel (VI): 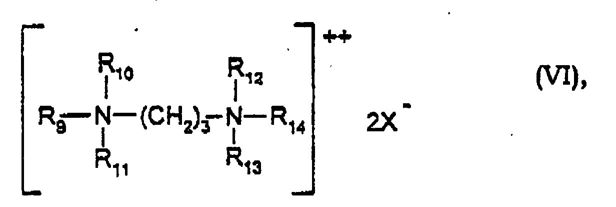
worin R₉ eine aliphatische Gruppe mit etwa 16 bis 30 Kohlenstoffatomen bedeutet, die Gruppen R₁₀, R₁₁, R₁₂, R₁₃ und R₁₄ unter Wasserstoff oder den Alkylgruppen mit 1 bis 4 Kohlenstoffatomen ausgewählt sind und X ein Anion ist, das unter den Halogeniden, Acetaten, Phosphaten, Nitrat und Methylsulfaten ausgewählt ist; und
- den quartären Ammoniumsalzen, die mindestens eine Estergruppe enthalten.

5. Nanoemulsion nach einem der Ansprüche 1 bis 4, **dadurch gekennzeichnet, daß** das kationische amphiphile Lipid in Konzentrationen von 1 bis 60 Gew.-%, bezogen auf das Gesamtgewicht der amphiphilen Lipidphase, und vorzugsweise 10 bis 50 Gew.-% vorliegt.

6. Nanoemulsion nach einem der Ansprüche 1 bis 5, **dadurch gekennzeichnet, daß** sie einen Ölanteil von 5 bis 40 Gew.-%, bezogen auf das Gesamtgewicht der Emulsion, enthält.

7. Nanoemulsion nach einem der Ansprüche 1 bis 6, **dadurch gekennzeichnet, daß** das Öl ausgewählt ist unter:
- den tierischen oder pflanzlichen Ölen, die von Estern von Fettsäuren und Polyolen gebildet werden, oder pflanzlichen oder tierischen Ölen der Formel R₉COOR₁₀, worin R₉ den Rest einer höheren Fettsäure mit 7 bis 29 Kohlenstoffatomen und R₁₀ eine geradkettige oder verzweigte Kohlenwasserstoffkette mit 3 bis 30 Kohlenstoffatomen bedeutet;
- den synthetischen oder natürlichen etherischen Ölen;
- Kohlenwasserstoffen;
- halogenierten kohlenstoffhaltigen Verbindungen;
- Estern von anorganischen Säuren und Alkoholen;
- Ethern und Polyethern; und
- Siliconen im Gemisch mit mindestens einem der oben definierten Öle.

8. Nanoemulsion nach einem der Ansprüche 1 bis 7, **dadurch gekennzeichnet, daß** sie einen wasserlöslichen oder fettlöslichen kosmetischen oder dermopharmazeutischen Wirkstoff enthält.

9. Zusammensetzung zur topischen Anwendung, **dadurch gekennzeichnet, daß** sie aus einer Nanoemulsion nach einem der Ansprüche 1 bis 8 besteht oder eine solche Nanoemulsion enthält.

10. Verwendung einer Nanoemulsion nach einem der Ansprüche 1 bis 9 als Produkt zur Pflege und/oder zum Waschen und/oder zum Schminken und/oder zum Abschminken des Körpers und/oder des Gesichts und/oder der Schleimhäute und/oder der Kopfhaut und/oder der Haare und/oder der Nägel und/oder der Wimpern und/oder der Augenbrauen oder in solchen Produkten.

11. Verfahren zur nicht therapeutischen Behandlung der Haut, der Haare, der Schleimhäute, der Nägel, der Wimpern, der Augenbrauen und/oder der Kopfhaut, **dadurch gekennzeichnet, daß** auf die Haut, die Haare, die Schleimhäute, die Nägel, die Wimpern, die Augenbrauen oder die Kopfhaut eine Nanoemulsion nach einem der Ansprüche 1 bis 9 aufgetragen wird.

12. Verfahren zur Herstellung einer Nanoemulsion nach einem der Ansprüche 1 bis 9, **dadurch gekennzeichnet, daß** die wäßrige Phase und die Ölphase unter kräftigem Rühren bei einer Umgebungstemperatur unter 45 °C vermischt werden und anschließend eine Hochdruckhomogenisierung bei einem Druck über 10⁸ Pa durchgeführt wird.

13. Verfahren nach Anspruch 12, **dadurch gekennzeichnet, daß** der Druck im Bereich von 12 · 10⁷ bis 18 · 10⁷ Pa liegt.

## Claims

1. Oil-in-water nanoemulsion whose oil globules have a mean size of less than 150 nm, comprising an amphiphilic lipid phase, **characterized in that** the said amphiphilic lipid phase comprises at least one nonionic amphiphilic lipid which is liquid at an ambient temperature of less than 45°C and at least one cationic amphiphilic lipid and **in that** the weight ratio of the quantity of oil to the quantity of amphiphilic lipid phase is between 2 and 10, and preferably between 3 and 6.

2. Nanoemulsion according to Claim 1, **characterized in that** the nonionic amphiphilic lipid is chosen from silicone surfactants and esters of at least one polyol chosen from the group formed by polyethylene glycol containing from 1 to 60 ethylene oxide units, sorbitan, glycerol containing from 2 to 30 ethylene oxide units, polyglycerols containing from 2 to 15 glycerol units, and of at least one fatty acid containing at least one saturated or unsaturated, linear or branched C₈-C₂₂ alkyl chain, and mixtures thereof.

3. Nanoemulsion according to either of Claims 1 and 2, **characterized in that** the cationic amphiphilic lipid is chosen from the group formed by quaternary ammonium salts and fatty amines.

4. Nanoemulsion according to Claim 3, **characterized in that** the quaternary ammonium salts are chosen from the group formed by:
- the quaternary ammonium salts of the following general formula (IV): 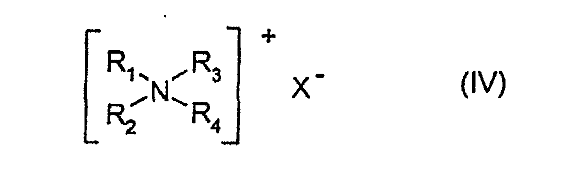 in which the radicals R₁ to R₄, which may be identical or different, represent a linear or branched aliphatic radical containing from 1 to 30 carbon atoms, or an aromatic radical such as aryl or alkylaryl. X is an anion chosen from the group comprising halides, phosphates, acetates, lactates, (C₂-C₆)alkyl sulphates and alkyl- or alkylarylsulphonates,
- the quaternary ammonium salts of imidazolinium,
- the quaternary diammonium salts of formula (VI): 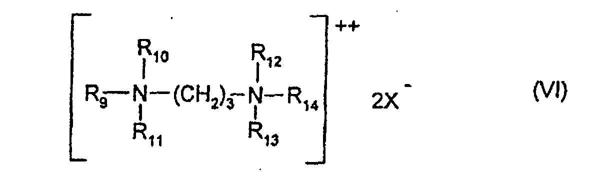 in which R₉ designates an aliphatic radical containing from about 16 to 30 carbon atoms, R₁₀, R₁₁, R₁₂, R₁₃ and R₁₄ are chosen from hydrogen or an alkyl radical containing from 1 to 4 carbon atoms, and X is an anion chosen from the group comprising the halides, acetates, phosphates, nitrates and methyl sulphates,
- the quaternary ammonium salts containing at least one ester functional group.

5. Nanoemulsion according to any one of Claims 1 to 4, **characterized in that** the cationic amphiphilic lipid is present in concentrations ranging from 1 to 60% by weight relative to the total weight of the amphiphilic lipid phase, and preferably from 10 to 50% by weight.

6. Nanoemulsion according to any one of Claims 1 to 5, **characterized in that** it comprises a proportion of oil ranging from 5 to 40% by weight relative to the total weight of the nanoemulsion.

7. Nanoemulsion according to any one of Claims 1 to 6, **characterized in that** the oil is chosen from the group formed by:
- animal or vegetable oils formed by esters of fatty acids and polyols or alternatively vegetable or animal oils of formula R₉COOR₁₀ in which R₉ represents the residue of a higher fatty acid containing from 7 to 29 carbon atoms and R₁₀ represents a linear or branched hydrocarbon chain containing from 3 to 30 carbon atoms;
- natural or synthetic essential oils;
- hydrocarbons;
- halogenated hydrocarbons;
- esters of an inorganic acid and of an alcohol;
- ethers and polyethers;
- silicones mixed with at least one of the oils defined above.

8. Nanoemulsion according to any one of Claims 1 to 7, **characterized in that** it comprises a watersoluble or fat-soluble cosmetic or dermopharmaceutical active agent.

9. Composition for topical use, **characterized in that** it consists of a nanoemulsion or comprises a nanoemulsion according to any one of Claims 1 to 8.

10. Use of a nanoemulsion as defined according to any one of Claims 1 to 9 as or in care and/or washing and/or make-up and/or make-up-removing products for the body and/or the face and/or the mucous membranes and/or the scalp and/or the hair and/or the nails and/or the eyelashes and/or the eyebrows.

11. Process for the nontherapeutic treatment of the skin, the hair, the mucous membranes, the nails, the eyelashes, the eyebrows and/or the scalp, **characterized in that** a nanoemulsion according to any one of Claims 1 to 9 is applied to the skin, the hair, the mucous membranes, the nails, the eyelashes, the eyebrows or the scalp.

12. Process for preparing a nanoemulsion as defined according to any one of Claims 1 to 9, **characterized in that** the aqueous phase and the oily phase are mixed, with vigorous stirring, at an ambient temperature of less than 45°C and **in that** a high-pressure homogenization is then carried out at a pressure greater than 10⁸ Pa.

13. Process according to Claim 12, **characterized in that** the pressure varies from 12 × 10⁷ to 18 × 10⁷ Pa.
